# EUROPÄISCHE PATENTANMELDUNG

(11) **EP 0 896 287 A1**
(43) Veröffentlichungstag der Anmeldung: **10.02.1999**
(21) Anmeldenummer: 98114563.4
(22) Anmeldetag: 03.08.1998
(51) Int. Cl.: G06F 19/00

(54) **Medizinische Systemarchitektur**

(30) Priorität: 04.08.1997 DE 19733743
(71) Anmelder: Siemens Health Services GmbH & Co KG, 91052 Erlangen (DE)
(72) Erfinder: Fuchs, Dieter, Dipl.-Ing. (FH), 91341 Röttenbach (DE)
(74) Vertreter: Epping, Wilhelm, Dr.-Ing.

(57) **Zusammenfassung**

Die Erfindung betrifft eine medizinische Systemarchitektur mit einer Modalität (1 bis 4) zur Erfassung von Bildern, einer Vorrichtung (5 bis 8, 11) zur Verarbeitung der Bilder und zur Aufnahme von Patientendaten, einer Vorrichtung (9) zur Übertragung der Bilder und einer Vorrichtung (10) zur Speicherung der Bilder und Patientendaten. An der Vorrichtung (9) zur Übertragung weiterhin ist eine Terminalstation (14) angeschlossen, über die in der Systemarchitektur gespeicherte Daten abrufbar sind, wobei die Terminalstation (14) ein Gehäuse (15) mit einem Display, einer Tastatur (16) einem Bildschirm (17) und/oder einem Touch-Screen (18) aufweist.

## Beschreibung

Die Erfindung betrifft eine medizinische Systemarchitektur mit einer Modalität zur Erfassung von Bildern, einer Vorrichtung zur Verarbeitung der Bilder und zur Aufnahme von patientenbezogenen Daten, einer Vorrichtung zur Übertragung der Bilder und patientenbezogenen Daten und einer Vorrichtung zur Speicherung der Bilder und der patientenbezogenen Daten.

Aus dem Buch "Bildgebende Systeme für die medizinische Diagnostik", herausgegeben von H. Morneburg, 3. Auflage, 1995, Seiten 684ff sind medizinische Systemarchitekturen bekannt, bei denen zum Abruf von Patientendaten und durch Modalitäten erzeugte Bilder Bildbetrachtungs- und Bildbearbeitungsplätze, sogenannte Workstations, an einem Bildkommunikationsnetz angeschlossen sind. Derartige Workstations stehen in speziellen Betrachtungsräumen und sind sehr aufwendig.

In der US 5,581,243 ist ein Computer beschrieben, bei dem auf einem Monitor als Eingabemittel ein Touch-Screen angebracht ist. Der Computer bildet ein geschlossenes System, bei dem über einen internen Bus der Datenaustausch mit den Peripheriegeräten erfolgt.

Die Erfindung geht von der Aufgabe aus, eine medizinische Systemarchitektur der eingangs genannten Art zu schaffen, die einen Abruf von Bildern oder Patientendaten auch außerhalb von Betrachtungsräumen, beispielsweise in Krankenstationen, ermöglicht.

Die Aufgabe wird erfindungsgemäß durch die Merkmale des Patentanspruches 1 gelost. Durch eine derartige Terminalstation, ein sogenannter Kiosk ähnlich einem Überweisungs- und Informationsautomaten von Banken, bei dem in einem Gerät alle Ein- und Ausgabemittel vereinigt sind, ist es dem Krankenhauspersonal möglich, an verschiedenen Orten, beispielsweise auch in Krankenstationen, wichtige, sofort gebrauchte Daten oder Untersuchungsergebnisse vor Ort abzurufen.

Erfindungsgemäß kann die Terminalstation als Ausgabemittel ein Display oder einen Bildschirm und als Eingabemittel eine Tastatur oder als Ein- und Ausgabemittel einen Touch-Screen aufweisen.

Dabei kann der Touch-Screen in mehrere Bereiche flexibel unterteilt sein, wobei in einem oberen Bereich ein Menübalken entsprechend der gewählten Funktion und/oder in einem unteren Bereich eine Tastatur einblendbar ist.

Die Eingabe kann sich vereinfachen, wenn an der Terminalstation als Eingabemittel ein Trackball angebracht ist. Die Daten lassen sich nur von berechtigten Personen abrufen, wenn die Terminalstation eine Vorrichtung zur Zugangskontrolle aufweist.

Der gerätetechnische sowie softwaremäßige Aufwand für die Terminalstation vereinfacht sich, wenn sie derart ausgebildet ist, daß in einem Server gespeicherte Software, beispielsweise JAVA-Programme, abrufbar ist.

Es hat sich als vorteilhaft erwiesen, wenn die Terminalstation zum Abruf der in der Systemarchitektur gespeicherten Bilder, Daten und Programme mit einem Mobilnetz über eine Handy- oder DECT-Schnittstelle verbunden ist.

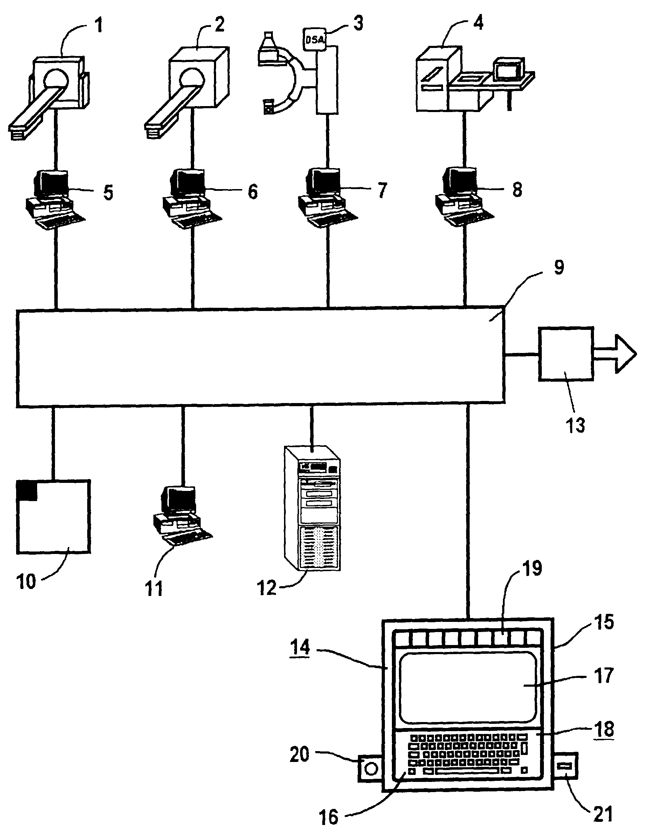

Die Erfindung ist nachfolgend anhand von in der Zeichnung dargestellten Ausführungsbeispielen näher erläutert.

In der Figur ist beispielhaft die Systemarchitektur eines medizinischen Bildkommunikationsnetzes dargestellt. Zur Erfassung medizinischer Bilder dienen die Modalitäten 1 bis 4, die als bilderzeugende Systeme beispielsweise eine CT-Einheit 1 für Computertomographie, eine MR-Einheit 2 für Magnetische Resonanz, eine DSA-Einheit 3 für digitale Subtraktionsangiographie und eine Röntgeneinheit 4 für die digitale Radiographie 4 aufweisen kann. An diese Modalitäten 1 bis 4 können Workstations 5 bis 8 angeschlossen sein, mit denen die erfaßten medizinischen Bilder verarbeitet und lokal abgespeichert werden können. Auch lassen sich zu den Bildern gehörende Patientendaten eingeben. Eine derartige Workstation ist beispielsweise ein sehr schneller Kleincomputer auf der Basis eines oder mehrerer schneller Prozessoren.

Die Workstations 5 bis 8 sind mit einem Bildkommunikationsnetz 9 zur Verteilung der erzeugten Bilder und Kommunikation verbunden. So können beispielsweise die in den Modalitäten 1 bis 4 erzeugten Bilder und die in den Workstations 5 bis 8 weiter verarbeiteten Bilder in zentralen Bildspeicher- und Bildarchivierungssystemen 10 abgespeichert oder an andere Workstations weitergeleitet werden.

An dem Bildkommunikationsnetz 9 sind weitere Workstations 11 als Befundungskonsolen angeschlossen, die lokale Bildspeicher aufweisen. In den Workstations 11 können die erfaßten und im Bildspeichersystem 10 abgelegten Bilder nachträglich zur Befundung abgerufen und in dem lokalen Bildspeicher abgelegt werden, von dem sie unmittelbar der an der Workstation 11 arbeitenden Befundungsperson zur Verfügung stehen können.

Weiterhin sind an dem Bildkommunikationsnetz 9 Server 12, beispielsweise Patientendaten-Server (PDS), Fileserver und/oder Programm-Server, angeschlossen.

Der Bild- und Datenaustausch über das Bildkommunikationsnetz 9 kann dabei nach dem DICOM-Standard erfolgen, einem Industriestandard zur Übertragung von Bildern und weiteren medizinischen Informationen zwischen Computern zur Ermöglichung der digitalen Kommunikation zwischen Diagnose- und Theraphiegeräten unterschiedlicher Hersteller. An dem Bildkommunikationsnetz 9 kann ein Netzwerk-Interface 13 angeschlossen sein, über das das interne Bildkommunikationsnetz 9 mit einem globalen Datennetz verbunden ist, so daß die standardisierten Daten mit unterschiedlichen Netzwerken weltweit ausgetauscht werden können.

Es kann aber auch das Bildkommunikationsnetz 9 mit dem Datennetz des Krankenhausinformationssystem verbinden, so daß auch weitere Patientendaten abrufbar sind.

Erfindungsgemäß ist an dem Bildkommunikationsnetz 9 eine Terminalstation 14 angeschlossen, über die in dem Bildspeichersystem 10 der Systemarchitektur gespeicherte Daten, beispielsweise Bild- oder Patientendaten, abrufbar sind. Die Terminalstation 14 besteht aus einem Gerät mit einem Gehäuse 15, das mit einer Tastatur 16 als Eingabemittel und einem Bildschirm 17 als Ausgabemittel versehen ist. Es kann aber auch erfindungsgemäß als Ein- und Ausgabemittel ein sogenannter Touch-Screen 18, ein Bildschirm 17 mit einem berührungsempfindlichen Vorsatz, verwendet werden, der mehrere Unterteilungen aufweisen kann, so daß die Tastatur 16 und der Bildschirm 17 von dem Touch-Screen 18 gebildet sein kann. Dazu wird im unteren Bereich des Touch-Screen 18 die Tastatur 16 eingeblendet. Der Touch-Screen 18 kann weiterhin im oberen Bereich einen Menübalken 19 aufweisen, durch den das gewünschte Program auswählbar ist, so daß von dem Server 12 beispielsweise die Java-Software geladen werden kann. Die Bedienung erfolgt dabei mittels auf dem Touch-Screen angezeigten Icons. Der Menübalken 19 kann flexibel unterteilt sein und nur die gerade zur Verfügung stehenden Befehle anzeigen. Der oder ein weiterer Menübalken kann auch im unteren Bereich eingeblendet werden, wenn beispielsweise die Tastatur 16 nicht benötigt wird.

Zur Vereinfachung der Bedienung der Terminalstation 14 kann diese weitere Eingabemittel, beispielsweise einen Trackball 20, aufweisen, mit dem ein Cursor auf dem Touch-Screen 18 bewegt werden kann und Eingaben auswählbar sind.

Weiterhin kann die Terminalstation 14 eine Vorrichtung zur Zugangskontrolle 21 aufweisen, mittels derer beispielsweise durch eine Magnetstreifenkarte gestaffelt nach Zugangserlaubnis die einzelnen Daten abrufbar sind.

Über die Terminalstation 14 lassen sich beispielsweise Bilder, Patientendaten, Befunde, Telefondienste und der elektronische Patienten-Record abfragen und aufrufen.

Auch ist eine mobile Anwendung beispielsweise mittels eines medizinischen Palmtops denkbar, bei der mit einem Mobilnetz über eine Handy- oder DECT-Schnittstelle Daten über eine Funkstrecke abrufbar sind.

Durch die erfindungsgemäße Terminalstation 14 der in Form eines Kiosks ähnlich einem Überweisungsautomaten von Banken aufgebaut ist, bei dem in einem Gerät alle Ein- und Ausgabemittel vereinigt sind, ist es dem Krankenhauspersonal möglich, an beliebigen Orten, beispielsweise auch in Krankenstationen, wichtige, sofort gebrauchte Daten, Bilder oder Untersuchungsergebnisse vor Ort abzurufen. Dabei zeichnet sich die Terminalstation 14 durch ihre einfache Bedienung aus.

## Patentansprüche

1. Medizinische Systemarchitektur mit einer Modalität (1 bis 4) zur Erfassung von Bildern, einer Vorrichtung (5 bis 8, 11) zur Verarbeitung der Bilder und zur Aufnahme von patientenbezogenen Daten, einer Vorrichtung (9) zur Übertragung der Bilder und patientenbezogenen Daten, einer Vorrichtung (10) zur Speicherung der Bilder, der patientenbezogenen Daten und von Programmen zur Wiedergabe der Bilder und Daten und einer an der Vorrichtung (9) zur Übertragung angeschlossenen Terminalstation (14), über die die in der Systemarchitektur gespeicherten Bilder, Daten und Programme abrufbar sind, wobei die Terminalstation (14) ein Gehäuse (15) mit Ein- und Ausgabemitteln (16 bis 20) aufweist.

2. Medizinische Systemarchitektur nach Anspruch 1, **dadurch gekennzeichnet,** daß die Terminalstation (14) als Ausgabemittel ein Display aufweist.

3. Medizinische Systemarchitektur nach Anspruch 1 oder 2, **dadurch gekennzeichnet,** daß die Terminalstation (14) als Eingabemittel eine Tastatur (16) und als Ausgabemittel einen Bildschirm (17) aufweist.

4. Medizinische Systemarchitektur nach Anspruch 1 oder 2, **dadurch gekennzeichnet,** daß die Terminalstation (14) als Ein- und Ausgabemittel einen Touch-Screen (18) aufweist.

5. Medizinische Systemarchitektur nach Anspruch 4, **dadurch gekennzeichnet,** daß der Touch-Screen (18) in mehrere Bereiche (16, 17, 19) unterteilt ist, wobei in einem oberen Bereich ein Menübalken (19) entsprechend der gewählten Funktion einblendbar ist.

6. Medizinische Systemarchitektur nach Anspruch 4 oder 5, **dadurch gekennzeichnet,** daß der Touch-Screen (18) in mehrere Bereiche (16, 17, 19) flexibel unterteilt ist, wobei in einem unteren Bereich eine Tastatur (16) einblendbar ist.

7. Medizinische Systemarchitektur nach einem der Ansprüche 1 bis 6, **dadurch gekennzeichnet,** daß an der Terminalstation (14) als Eingabemittel ein Trackball (20) angebracht ist.

8. Medizinische Systemarchitektur nach einem der Ansprüche 1 bis 7, **dadurch gekennzeichnet,** daß die Terminalstation (14) eine Vorrichtung (21) zur Zugangskontrolle aufweist.

9. Medizinische Systemarchitektur nach einem der Ansprüche 1 bis 8, **dadurch gekennzeichnet,** daß die Terminalstation (14) derart ausgebildet ist, daß in einem Server (12) gespeicherte Software abrufbar ist.

10. Medizinische Systemarchitektur nach einem der Ansprüche 1 bis 9, **dadurch gekennzeichnet,** daß die Terminalstation (14) derart ausgebildet ist, daß in einem Server (12) gespeicherte JAVA-Programme abrufbar sind.

11. Medizinische Systemarchitektur nach einem der Ansprüche 1 bis 9, **dadurch gekennzeichnet,** daß die Terminalstation (14) zum Abruf der in der Systemarchitektur gespeicherten Bilder, Daten und Programme mit einem Mobilnetz über eine Handy- oder DECT-Schnittstelle verbunden ist.
